(19) **Europäisches Patentamt**

**European Patent Office**

**Office européen des brevets**

(11) **EP 0 737 731 B1**

(12) **EUROPEAN PATENT SPECIFICATION**

(45) Date of publication and mention
of the grant of the patent:
**08.11.2000 Bulletin 2000/45**

(51) Int Cl.[7]: **C09J 7/02**, B65B 63/02,
A61F 13/15, B32B 27/32,
B65D 65/40

(21) Application number: **96105802.1**

(22) Date of filing: **12.04.1996**

(54) **Separator, packaging material, or pressure-sensitive adhesive tape base material**

Abscheider, Verpackungsmaterial oder druckempfindliches Klebebandträgermaterial

Séparateur, matériau d'emballage ou matériau de base pour ruban adhésif sensible à la presion

(84) Designated Contracting States:
**DE FR GB**

(30) Priority: **13.04.1995 JP 8825995**
**06.02.1996 JP 1991896**

(43) Date of publication of application:
**16.10.1996 Bulletin 1996/42**

(73) Proprietor: **NITTO DENKO CORPORATION**
**Osaka (JP)**

(72) Inventors:
• **Arakawa, Masaaki**
**Ibaraki-shi, Osaka (JP)**
• **Goto, Kazuhito**
**Ibaraki-shi, Osaka (JP)**

• **Uozumi, Hideaki**
**Ibaraki-shi, Osaka (JP)**
• **Ito, Takio**
**Ibaraki-shi, Osaka (JP)**

(74) Representative: **Grünecker, Kinkeldey,**
**Stockmair & Schwanhäusser Anwaltssozietät**
**Maximilianstrasse 58**
**80538 München (DE)**

(56) References cited:
**EP-A- 0 652 102      WO-A-94/24977**

Remarks:
The file contains technical information submitted
after the application was filed and not included in this
specification

**Description**

FIELD OF THE INVENTION

The present invention relates to a separator for pressure-sensitive adhesive articles such as a pressure-sensitive adhesive sheet, a (double-sided) pressure-sensitive adhesive tape, a tack, etc., and for disposable articles such as a paper diaper, a sanitary napkin. etc., or a packaging material being used for packing foods, pressure-sensitive adhesive articles such as pressure-sensitive adhesive tacks, etc., and sanitary articles such as a sanitary napkin, etc. In particular, the separator of the present invention is suitably used as a separator for a disposable diaper and a sanitary napkin and also the packaging material of the present invention is suitably used as a package for a sanitary napkin. Furthermore, the present invention relates to a base material for a pressure-sensitive adhesive tape useful in medical field or for surface protection.

BACKGROUND OF THE INVENTION

Hitherto, as separators for disposable articles such as disposable diapers and napkins and as packaging materials for sanitary articles such as sanitary napkins, etc., various materials are used. For example, as a packaging material for a napkin, a releasing tape and a packaging material each comprising a substrate composed of a flexible plastic film having thereon a releasing agent layer are proposed as described, e.g., in JP-A-3-24949 and JP-A-3-184543 (the term "JP-A" as used herein means an "unexamined published Japanese patent application"). In many cases, the substrates used for the foregoing materials are plastic films composed of a single component such as polyethylene, polypropylene, polyester, nylon, cellophane, etc., and the plastic films usually contain additive ingredients such as an antioxidant, an antistatic agent, a lubricant, and an ultraviolet absorber.

In the use of the plastic films composed of a single component, there is a problem that the substrate is too soft or, to the contrary, too hard. That is, if the substrate is too soft, the substrate is stretched or cut in the conveying step in the production line of napkins, etc., which results in greatly lowering the productivity. Also, if the substrate is too hard, the product such as the releasing tape and the packaging material has a disadvantage that a soft feeling from the point of the practical use is lost and the releasing sound becomes large to lower the commercial value thereof.

In the case of using such a packaging material as a separator or a separator-less package in a production line for producing sanitary napkins or the like, the method generally used especially from the standpoint of production efficiency is to directly apply a heated hot-melt pressure-sensitive adhesive to the separator or the packaging material and transfer the applied pressure-sensitive adhesive to a napkin body. Consequently, if the substrate of the separator or the packaging material is soft and has poor heat resistance, it disadvantageously melts or suffers wrinkling due to heat, making the production difficult.

Although it has hence been necessary to use paper or a rigid or thick plastic film as a substrate having improved heat resistance, such a substrate has not been put to practical use because it has poor pliability, makes a noise when crumpled, and leads to a cost increase.

There has been another problem that if a silicone releasing agent is applied to a surface of a plastic film containing additive ingredients such as an antioxidant, an antistatic agent, a lubricant, and an ultraviolet absorber so as to form a releasing agent layer, the additive ingredients which have migrated to the surface of the plastic film inhibit the curing reaction of the silicone releasing agent and the resulting insufficiently cured silicone releasing agent layer reduces the adhesion strength of the pressure-sensitive adhesive layer to be in contact therewith.

EP-A-0652102 describes a separator, a packaging material and a pressure-sensitive adhesive tape base material. WO-A-94/24977 describes a pressure-sensitive adhesive tape comprising a substrate and a silicone releasing aging layer.

SUMMARY OF THE INVENTION

As a result of intensive studies made by the present inventors in order to overcome the problems described above, it has been found that a specific thermal analysis is most suitable for the selection of a practical substrate for use in the present invention, and that a substrate which has specific values when examined by this analysis can be the substrate of an extremely practical separator or packaging material free from the drawbacks described above.

It has also been found that in the case of a separator or the like comprising a plastic film substrate mainly composed of a thermoplastic resin and a silicone releasing agent layer formed on the substrate, the problem that the adhesion strength of a pressure-sensitive adhesive layer to be in contact with the silicone releasing agent layer is reduced can be prevented by regulating the contents of specific additives in the plastic film to a specific amount or below.

The object of the invention, to overcome the above problems, is achieved by the subject matter of claim 1. Embodiments of the invention are indicated in the subclaims.

## BRIEF DESCRIPTION OF THE DRAWINGS

Fig. 1 is a sectional view showing an embodiment of the separator or packaging material according to the present invention.

Fig. 2 is a sectional view showing another embodiment of the separator or packaging material according to the present invention.

Fig. 3 is a sectional view showing still another embodiment of the separator or packaging material according to the present invention.

Fig. 4 is a sectional view showing a further embodiment of the separator or packaging material according to the present invention.

Fig. 5 is sectional view showing an embodiment of a pressure-sensitive adhesive tape employing the pressure-sensitive adhesive tape base material according to the present invention.

Fig. 6 is a diagrammatic view of a sanitary napkin package produced using the packaging material of the present invention.

Fig. 7 is a diagrammatic view of a sanitary napkin package produced using the separator of the present invention.

Fig. 8 is a diagrammatic view of another sanitary napkin package produced using the separator of the present invention.

Description of Symbols

1:       substrate
11:     matte surface or embossed surface
2:       releasing agent layer
3:       pressure-sensitive adhesive layer
A:      package
B:      sanitary napkin
C:      separator
D:      bag
E:      pressure-sensitive adhesive tape base material

## DETAILED DESCRIPTION OF THE INVENTION

The thermomechanical analysis (thermomechanometry) employed in the present invention is a technique for measuring deformations of a sample in which a non-oscillatory load is imposed on the sample while the temperature of the sample is changed according to a program. The optimal measurement mode (measurement method) can be selected according to the shape of the sample and the purpose of the measurement. In the present invention, the extension mode which will be described later is especially employed. This thermomechanical analysis is particularly effective and practically advantageous especially in determining the thermal shock which the substrate of the present invention receives upon contact with a hot-melt pressure-sensitive adhesive, because the analysis reveals the heat distortion temperature (softening temperature) and fluidization initiation temperature of the sample.

The substrate for use in the present invention is characterized in that the average coefficient of linear expansion thereof (dimensional change per unit length of the material upon a temperature change of 1°C) calculated using the following equation 1 from thermomechanical analysis data is regulated to values in specific ranges.

Equation 1:

$$\beta_s \ (T_1 \sim T_2) = \frac{1}{L_0 \times 10^3} \cdot \frac{\Delta l_s \ (T_2) - \Delta l_s \ (T_1)}{T_2 - T_1}$$

$T$ :               Temperature (°C)
$\beta_s(T_1\text{-}T_2)$:       Coefficient of linear expansion (°C$^{-1}$) of the sample in the range of from $T_1$ to $T_2$
$L_0$ :             Length (mm) of the sample
$\Delta l_s \ (T)$ :        Found value ($\mu$m) determined by the thermomechanical analysis at a sample temperature of $T$

It is crucially important in the present invention that the average coefficient of linear expansion $(1/°Cx10^{-3})$ in the machining direction calculated using the above equation for each temperature range should be as follows:

70-80°C : ≤0.7, preferably 0.1-0.6
80-90°C : ≤0.9, preferably 0.3-0.8
90-100°C: ≤1.4, preferably 0.3-1.3
100-110°C: ≤4.0, preferably 0.5-3.9,

while that in the traversing direction for each temperature range should be as follows:

70-80°C : ≤1.3, preferably 0.3-1.2
80-90°C : ≤1.3, preferably 0.5-1.2
90-100°C: ≤2.2, preferably 0.7-2.1
100-110°C: ≤5.2, preferably 1.0-5.1.

If the average coefficients of linear expansion in the machining direction and traversing direction for each temperature range exceed the values specified above, there is a problem that the thin film develops wrinkles due to thermal expansion and contraction upon the application of a heated hot-melt pressure-sensitive adhesive in a process for producing sanitary napkins or the like, resulting in a poor appearance.

In the present invention, the releasing agent layer which will be described later is formed on part or the whole of one or both sides of a substrate having the above-described property, whereby a separator, a packaging material, a pressure-sensitive adhesive tape base material, or the like which each has improved releasing properties can be obtained. This releasing agent layer serves to protect a pressure-sensitive adhesive layer. In the case where the substrate is partly coated with the releasing agent, this treatment also produces the effect of enabling the control of peeling force.

In the present invention, the sound pressure level measured by the method described in Examples given later is preferably 80 dB or lower, more preferably from 60 to 70 dB.

Due to this regulation of sound pressure level, in the case where the separator or packaging material of the present invention is used as a separator or package for a sanitary napkin, they make a diminished noise, for example, when they are peeled from the sanitary napkin, when the separator is folded by hand, or when the packaging bag is torn. Thus, the separator or package can secure female privacy and the article is of extremely high value.

The thickness of the substrate is not particularly limited. Although the thickness thereof is usually 500 μm or smaller, it is preferably from 10 to 200 μm, more preferably from 10 to 50 μm, from the standpoints of softness and pliability.

Since the substrate for use in the present invention has specific values of the average coefficient of linear expansion as described above, the substrate can have sufficient heat resistance and strength in a production line even when it is relatively thin.

From the standpoint of traveling through a production line, the separator, packaging material, or pressure-sensitive adhesive tape base material of the present invention should have some degree of strength. Usually, the tensile strength thereof in the machining direction is generally 300 gf/10 mm (2.94 N/10 mm) or higher, preferably from 300 to 1,200 gf/10 mm (2.94 to 11.76 N/10 mm), and most desirably from 300 to 500 gf/10 mm (2.94 to 4.90 N/10 mm), while the tensile strength thereof in the traversing direction is generally 200 gf/10 mm (1.96 N/10 mm) or higher, preferably from 200 to 1,050 gf/10 mm (1.96 to 10.29 N/10 mm), and most preferably from 300 to 500 gf/10 mm (2.94 to 4.90 N/10 mm).

The material of the substrate of the separator, packaging material, or pressure-sensitive adhesive tape base material of the present invention is not particularly limited as long as the substrate has the specific values of the average coefficient of linear expansion as given hereinabove. However, the substrate in the present invention preferably comprises a plastic film comprising a thermoplastic polyolefin resin as a main component.

Examples of the thermoplastic polyolefin resin include polyethylene, polybutene, polyhexene, and polyoctene. Such polyolefins can be used alone or as a mixture thereof. Especially preferably used is polyethylene. The polyethylene may be any one of ultralow-density, low-density, linear low-density, medium-density, and high-density polyethylenes, or may be a blend of any two or more thereof. Especially preferred examples of the thermoplastic polyolefin resin have a polyethylene content of 60% by weight or higher. If desired, polypropylene is added preferably in an amount of 40% by weight or smaller to improve heat resistance. An elastomer ingredient such as a styrene type, polyolefin type, or polyester type may be added preferably in an amount of 40% by weight or smaller to improve pliability. If desired, a multilayered film substrate composed of two or more layers can be used.

Examples of the polymer constituting the thermoplastic plastic film include polyethylene, polypropylene, an ethylene-vinyl acetate copolymer, an ethylene-(meth)acrylic acid copolymer, an ethylene-methyl (meth)acrylate copolymer, an ethylene-ethyl (meth)acrylate copolymer, a polyethylene-propylene copolymer, an olefinic elastomer, a styrenic elastomer, polyisobutyrene, butyl rubber, and a composite resin composed of two or more selected from these resins. In this case, as the olefinic elastomer, there are an ethylene-propylene rubber (EPT), an ethylene-propylene-diene rubber (EPDM), an ethylene-propylene series elastomer, an ethylene-butene series elastomer, etc. As the styrenic elastomer, there are a styrene-isoprene-styrene block copolymer (SIS), a styrene-butadiene-styrene block copolymer (SBS), etc., and the hydrogenated products of them.

In the present invention, the substrate is preferably composed of at least two layers in which one layer is in contact on one or each side with a heat-sealable layer having a melting point lower by at least 5°C, preferably by from 10 to 60°C, than the melting point of that one layer. This layer constitution is advantageous in that heat sealing can be easily conducted without fail, and is suitable especially when the material of the present invention is formed into a bag or is fixed to a substance.

The reason for the above is as follows. When a single-layer substrate is heat-sealed, a high sealing temperature may cause a hole in the sheet and temperature control for avoiding this trouble is not easy. In contrast, a substrate composed of two or more layers including a layer suitable for heat sealing has the effect of facilitating the selection of heat-sealing conditions.

Examples of the multilayered substrate include a two-layer substrate composed of an upper layer made of high-density polyethylene (melting point, 120°C) and a lower layer (heat-sealable layer) made of low-density polyethylene (melting point, 100°C).

In the present invention, use of a blend of two or more of the polymers enumerated above can give a substrate having a rough surface such as a matte surface or an embossed surface. The effects brought about by this rough surface are that the substrate has a matte appearance to give a feeling of high grade, and that the substrate has a reduced coefficient of dynamic friction and hence improved running properties in a production line. It is thought that in blending polymers having poor compatibility with each other, the polymers which are being melt-mixed constitute separate phases due to a difference in surface tension during melting and hence give a non-homogeneous mixture having not a smooth but a rough surface, i.e., a matte or embossed surface. This surface desirably has a roughness of usually from 2 to 30 $\mu$m, preferably from 2 to 5 $\mu$m, in terms of the value of $R_a$ determined by the method described later. In the case where a substrate film is embossed or matted by means of, e.g., a roll as described later, the resulting rough surface preferably has a value of $R_a$ within a half of the film thickness. The blending of polymers having different properties can give a blend possessing desirable properties of the polymers and not having the undesirable properties thereof. Thus, polymer blending has the effect of giving a polymer having a new function, e.g., combining pliability and nerve.

Blending a relatively soft polymer or elastomer to produce a substrate also has the effect that the peeling noise made by, e.g., peeling off the substrate can be diminished to such a level that the use thereof is not noticed by other persons.

Besides the polymer blending method described above, another usable method for positively forming a matte or embossed surface is to form the surface roughness by means of the surface of a roll, for example, after T-die extrusion during film production. In the present invention, the latter method is preferred in forming an embossed surface.

The plastic film for use as a substrate in the present invention can have a multilayer structure composed of, e.g., two, three, or four layers, and at least one side of this multilayered plastic film may be constituted of a layer made of the above-described polymer blend and having a matte or embossed surface. Like polymer blending, this multilayer constitution has an advantage that properties of the individual polymer layers can be combined to impart a new function, e.g., a combination of an improvement in the heat sealability as described above or in tear strength and a satisfactory feeling.

This multilayered plastic film is not particularly limited in any layer other than the polymer blend layer having a matte or embossed surface. When the layer other than the polymer blend layer is a heat sealing layer, it is preferred that the layer is composed of polyethylene, polypropylene, an ethylene-vinyl acetate copolymer, an ethylene-(meth)acrylic acid copolymer, an ethylene-methyl (meth)acrylate copolymer, an ethylene-ethyl (meth)acrylate copolymer, a polyethylene-propylene copolymer, an olefinic elastomer, a styrenic elastomer, polyisobutyrene, butyl rubber, and a composite resin composed of two or more selected from these resins.

Another embodiment of the substrate for use in the present invention comprises a film made of a plastic which has a matte or embossed surface having a surface roughness ($R_a$) of about a half of the film thickness or smaller on at least one side of the film and which has a releasing agent layer on the other side. This constitution has effects of softness to the touch, a high-grade appearance, and improved running properties in a production line (a reduced coefficient of dynamic friction).

Still another embodiment comprises a plastic film which has a matte or embossed surface having a surface roughness ($R_a$) of a half of the film thickness or smaller on at least one side of the film and which has a releasing agent layer on the matte or embossed surface.

In this case, the coefficient of static friction of the matte or embossed surface which has been coated with a releasing agent is generally less than 1.5, preferably from 0.4 to 1.0, and the coefficient of dynamic friction thereof is generally less than 1.5, preferably from 0.2 to 1.3. This regulation of the coefficients of friction has the effect that the substrate has improved running properties in a production line and is less apt to wrinkle, meandering, or break when in contact with rolls.

In the present invention, forming a releasing agent layer on part or the whole of both sides or one side of the substrate gives a separator, a packaging material, a pressure-sensitive adhesive tape base material, or the like.

For forming the releasing agent layer, use may be made of a long-chain alkyl releasing agent (e.g., a polymer of an alkyl acrylate in which the alkyl is a long-chain alkyl having 12 or more carbon atoms and showing releasing properties; a copolymer of a long-chain alkyl acrylate and another vinyl monomer; or a reaction product obtained by reacting poly(vinyl alcohol) with a long-chain alkyl ingredient such as a long-chain alkyl isocyanate) or a silicone releasing agent. The silicone releasing agent can be suitably selected from heat-curing, ultraviolet-curing, electron beam-curing, and other silicone releasing agents, but an especially preferred silicone releasing agent is of the ultraviolet-curing or electron beam-curing type. This releasing agent layer may be formed either over the whole surface of the film or on part of the surface (e.g., on both dry edges, in a patterned area, or in a central area only). In the case where an ultraviolet-curing or electron beam-curing silicone releasing agent is used for the releasing agent layer, the kinds of such a silicone releasing agent are not limited, and known silicone releasing agents can be used. For curing the ultraviolet-curing silicone releasing agent, ultraviolet ray having a wavelength of 400 nm or less can be generally used, and for instance, a medium or high pressure mercury lamp can be used. For curing the electron beam-curing silicone releasing agent, a low pressure electron beam accelerator and the like can be used.

The thickness of the releasing agent layer formed on the substrate is preferably from 0.05 to 4.0 $\mu$m, more preferably from 0.3 to 1.5 $\mu$m. If a releasing agent layer having a thickness smaller than 0.05 $\mu$m is to be formed, it is not easy to evenly apply a releasing agent to the substrate surface and unevenness of coating (pinholes) tends to result. Even if the thickness of the coating exceeds 4.0 $\mu$m, further improvement of releasing property is not much exhibited. The provision of such a thick releasing agent layer is economically undesirable.

In the case where a silicone releasing agent is used for forming the releasing agent layer in the present invention, the content of an antioxidant, an antistatic agent, a lubricant, and an ultraviolet absorber (these being hereinafter often referred to as "additive ingredients") in the plastic film substrate comprising a thermoplastic resin as a main component each is in an amount of 0,01-0,4 % by weight. If the content of any of these additive ingredients exceeds 0.4% by weight, the curing reaction of the silicone releasing agent is apt to be inhibited and this tends to result in a problem that the silicone releasing agent layer reduces the adhesion strength of the pressure-sensitive adhesive layer to be in contact therewith. The additive ingredients may be suitably incorporated into the plastic film each in an amount within the range specified above, but the preferred range of the content of each additive ingredient is from 0.01 to 0.3% by weight.

In the case of using an antioxidant, known antioxidants conventionally incorporated for the inhibition of the oxidative deterioration of plastic films can be used, and examples thereof include aromatic amine derivatives and phenol derivatives. Specific examples of such antioxidants include 2,6-di-t-butyl-p-cresol, tetrakis[3-(4-hydroxy-3,5-di-t-butylphenyl)propionyloxymethyl]methane, dilauryl thiodipropionate, distearylpentaerythritol diphosphite, tetrakis (2,4-di-t-butylphenyl)-4,4'-biphenylene diphosphite, Irganox 1010 (manufactured by Ciba Geigy Ltd.), Sumilizer BHT (manufactured by Sumitomo Chemical Co., Ltd., Japan), Yoshinox DLTP (manufactured by Yoshitomi Pharmaceutical Industries, Ltd., Japan), Weston 618 (manufactured by G.E.), and Sandostab P-EPQ (manufactured by Sandoz Ltd.).

In the case of using an antistatic agent, known antistatic agents can be used, and examples thereof include nonionic polyoxyethylene alkylamine antistatic agents (Armostat 310, manufactured by Lion Corp., Japan), anionic antistatic agents, and cationic antistatic agents.

In the case of using a lubricant, known lubricants conventionally incorporated for improving the fluidity of plastic films can be used, and examples thereof include organic fatty acids and salts thereof. Specific examples of such lubricants include stearamide, oleamide, elucamide, Amide S (manufactured by Nitto Chemical Industry Co., Ltd., Japan), and Armoslip CP and Armoslip E (manufactured by Lion Corp.).

In the case of using an ultraviolet absorber, known ultraviolet absorbers conventionally incorporated for inhibiting plastic films from being deteriorated by light (ultraviolet) can be used, and examples thereof include benzophenone compounds, salicylic ester compounds, benzotriazole compounds, and acrylonitrile compounds. Specific examples of such ultraviolet absorbers include Sumisorb 300, manufactured by Sumitomo Chemical Co., Ltd.

The above-described plastic film which has been regulated so that the contents of the specific additive ingredients do not exceed the specific amount can be produced by adding an antioxidant, an antistatic agent, a lubricant, and an ultraviolet absorber to a thermoplastic resin as the main ingredient according to need in respective amounts which satisfy the content requirement specified in the present invention, and then forming the mixture into a film by a known film-forming technique.

There are no particular limitations on additives (e.g., fillers and pigments) other than the specific additive ingredients described above. Known such additives can be incorporated in ordinary amounts.

In the case where the substrate of the present invention is used as a pressure-sensitive adhesive tape base material, the kind of the pressure-sensitive adhesive is not limited in any way. Preferred examples of the method of coating the adhesive on the base material include a method of transferring an ordinary acrylic or rubber type pressure-sensitive adhesive by laminating or a method of directly applying such a pressure-sensitive adhesive to the substrate by the curtain flow or melt flow technique, with which the substrate is less heated.

Embodiments of the present invention will be explained below by reference to the drawings.

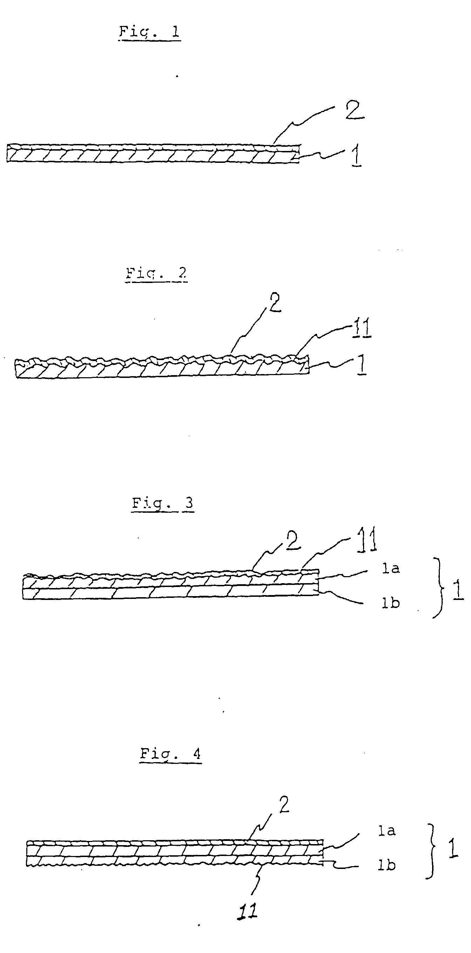

Figs. 1 to 4 each is a sectional view showing an embodiment of the separator according to the present invention. The embodiment shown in Fig. 1 is composed of a substrate 1 comprising a plastic film mainly composed of a thermoplastic resin and a releasing agent layer 2 formed on one side of the substrate 1. The embodiment shown in Fig. 2 is composed of a substrate 1 which comprises a plastic film mainly composed of a thermoplastic resin and has on one side a matte surface (or embossed surface) 11, and a releasing agent layer 2 formed on the matte surface 11.

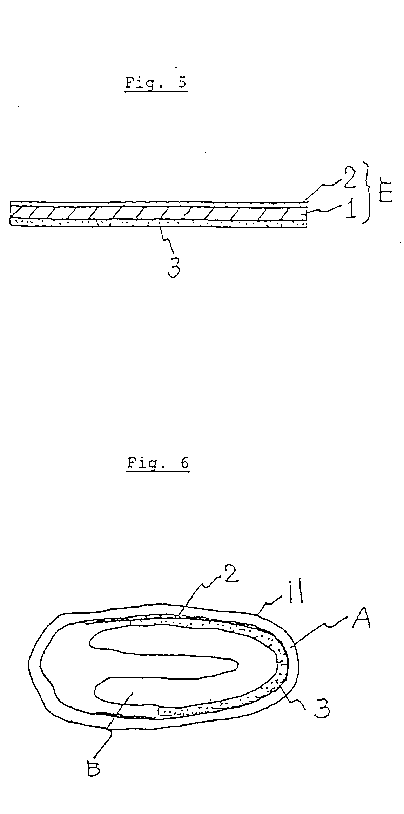

The embodiment shown in Fig. 3 is composed of a substrate 1 which comprises a two-layer plastic film mainly composed of thermoplastic resins (first layer 1a and second layer 1b) and in which the first layer 1a has a matte surface (or embossed surface) 11, and a releasing agent layer 2 formed on the matte surface 11. The embodiment shown in Fig. 4 is composed of a substrate 1 which comprises a two-layer plastic film mainly composed of thermoplastic resins (first layer 1a and second layer 1b) and in which the second layer 1b has a matte surface (or embossed surface) 11, and a releasing agent layer 2 formed on the surface of the first layer 1a. Fig. 5 shows a pressure-sensitive adhesive tape which is composed of a pressure-sensitive adhesive tape base material E according to the present invention composed of a substrate 1 having a releasing agent layer 2 formed on one side thereof, and a pressure-sensitive adhesive layer 3 formed on the other side of the base material E.

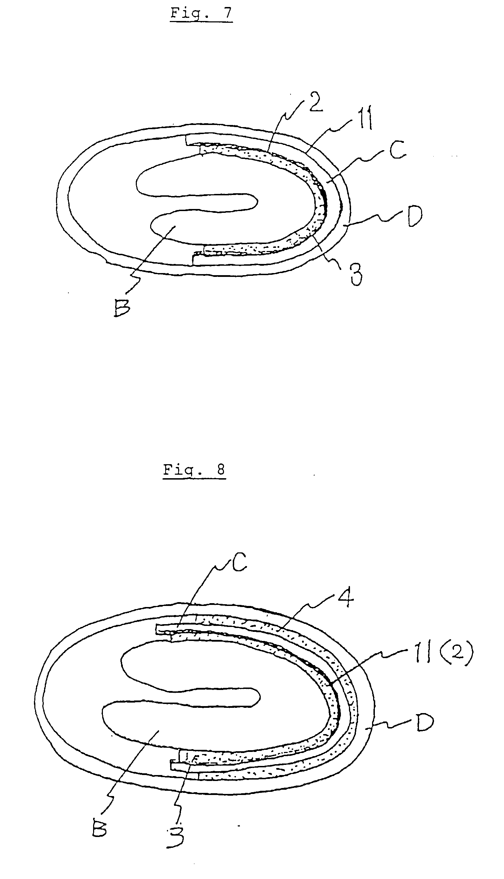

Figs. 6 to 8 each is a diagrammatic view of a sanitary napkin package as an application example of the separator or packaging material of the present invention. In Fig. 6, symbol A denotes the package comprising a packaging material according to the present invention. This bag A has a matte surface 11 on the outer side and has a releasing agent layer 2 on the inner side, facing a pressure-sensitive adhesive layer 3. In this bag A is contained a sanitary napkin B, which is fixed through the pressure-sensitive adhesive layer 3. The packages shown in Figs. 7 and 8 each comprises a sanitary napkin B which has a pressure-sensitive adhesive layer 3 protected with a separator C according to the present invention, and which is contained in a bag D. In Fig. 7, the separator C has a matte surface 11 on the outer side and has a releasing agent layer 2 on the inner side, i.e., the side facing the pressure-sensitive adhesive layer 3. In Fig. 8, the separator C is fixed to and contained in the bag D through another pressure-sensitive adhesive layer (e.g., a hot-melt pressure-sensitive adhesive layer) 4. Heat sealing may be used for fixing. In this package, the separator C has a matte surface 11 on the inner side, i.e., the side facing the pressure-sensitive adhesive layer 3, and has a releasing agent layer 2 on this side.

The present invention will be explained below in more detail by reference to Examples and Comparative Examples.

EXAMPLES 1 TO 3

Films each having the composition and the thickness given in Table 1 were formed by T-die extrusion. On one side of each film, an ultraviolet-curing silicone was coated and irradiated with ultraviolet ray by means of a high pressure mercury lamp to thereby form a releasing agent layer having a thickness of 1 μm. Thus, the substrate samples according to the present invention were obtained.

EP 0 737 731 B1

Table 1

| | Example | | | Comparative Example | | |
|---|---|---|---|---|---|---|
| | 1 | 2 | 3 | 1 | 2 | 3 |
| Composition (wt%) | | | | | | |
| Low-density polyethylene | 98 | 97 | 50 | 90 | 97 | 10 |
| High-density polyethylene | 0.5 | − | − | − | − | − |
| Polybutene | − | − | − | − | 0.5 | − |
| Polyhexene | 1 | 2 | − | 3 | 0.5 | − |
| Polyoctene | 0.5 | 1 | − | 2 | 0.5 | − |
| Polypropylene | − | − | 20 | − | 1.5 | 70 |
| Ethylene-vinyl acetate copolymer | − | − | 20 | − | − | − |
| Poly 4-Methylpentene-1 | − | − | − | 5 | − | − |
| EPDM | − | − | 10 | − | − | 20 |
| Thickness (μm) | 34 | 25 | 40 | 28 | 12 | 40 |

The thus-obtained samples were examined by the following thermomechanical analysis, and the results of the determination of the average coefficients of linear expansion are shown in Table 2.

Thermomechanical Analysis

Apparatus: SEIKO TMA100
Measurement method: extension mode
Sample shape: 10 mm (length) x 3 mm (width)
Measurement conditions: heating rate, 3°C/min; load, 2 g
Calculation of the average coefficient of linear expansion: by the method using equation 1 given hereinabove.

## Table 2

| | | Example | | | Comparative Example | | |
|---|---|---|---|---|---|---|---|
| | | 1 | 2 | 3 | 1 | 2 | 3 |
| Average coefficient of linear expansion | | | | | | | |
| 70-80°C | Machining direction | 0.5 | 0.3 | 0.6 | 0.9 | 0.7 | 0.9 |
| | Traversing direction | 0.5 | 0.8 | 1.1 | 1.4 | 1.1 | 1.4 |
| 80-90°C | Machining direction | 0.6 | 0.5 | 0.8 | 1.0 | 0.9 | 1.2 |
| | Traversing direction | 0.7 | 1.0 | 1.1 | 1.9 | 1.2 | 2.0 |
| 90-100°C | Machining direction | 1.0 | 0.5 | 1.1 | 1.7 | 1.2 | 1.5 |
| | Traversing direction | 1.0 | 1.5 | 1.9 | 2.7 | 2.0 | 2.2 |
| 100-110°C | Machining direction | 2.5 | 1.3 | 3.7 | 9.6 | 3.8 | 4.0 |
| | Traversing direction | 3.0 | 4.1 | 5.0 | * | 7.6 | 5.2 |

unit: $1/°C \times 10^{-3}$      *: melted

Other properties were examined by the following methods. The results obtained are shown in Table 3.

Sound Pressure Level

Each sound pressure was measured with an A-level precision noise meter placed at a distance of 100 mm from the sound source under the following conditions. The results obtained are shown in Table 3.

(1) Tearing noise: A sample having dimensions of 100 mm by 100 mm was notched at the center thereof. The notched sample was torn apart at a rate of about from 300 mm/min to 50 m/min, and the resulting sound pressure was measured.
(2) Crushing noise: Two samples each having dimensions of 100 mm by 100 mm were superposed and formed into a roll. This roll was crushed at a rate of about 300 mm/min, and the resulting sound pressure was measured.
(3) Noise made by crumpling by hand: Two samples each having dimensions of 100 mm by 100 mm were superposed and crumpled by hand, and the resulting sound pressure was measured.

Tensile Strength

A sample having a length of 50 mm and a width of 10 mm was tested with a tensile tester at a pulling rate of 300 mm/min, and the strength at break of the sample was measured. The results obtained are shown in Table 3.

Softness

Each sample was actually touched by ten persons, and the feel was evaluated based on the following criteria. The results obtained are shown in Table 3.

A : From seven to ten persons judged satisfactory (soft).
B : From three to six persons judged satisfactory (soft but slightly rigid).
C : Two or fewer persons judged satisfactory (rigid).

Table 3

| | Example | | | Comparative Example | | |
|---|---|---|---|---|---|---|
| | 1 | 2 | 3 | 1 | 2 | 3 |
| Sound pressure level, dB | | | | | | |
| Tearing noise | 67 | 62 | 68 | 68 | 62 | 83 |
| Crushing noise | 68 | 63 | 72 | 68 | 62 | 82 |
| Noise made by clumping by hand | 69 | 69 | 78 | 69 | 69 | 93 |
| Tensile strength | | | | | | |
| Machining direction (gf/10mm) | 390 | 500 | 320 | 290 | 230 | 800 |
| " (N/10mm) | 3.82 | 4.90 | 3.14 | 2.84 | 2.26 | 7.85 |
| Traversing direction (gf/10mm) | 380 | 480 | 200 | 180 | 150 | 920 |
| " (N/10mm) | 3.73 | 4.71 | 1.96 | 1.77 | 1.47 | 9.02 |
| Softness | A | A | B | A | A | C |

Table 3 shows the following. The samples of the Examples were satisfactory in all the properties. In contrast, the samples of Comparative Examples 1 and 2 had an insufficient strength and hence had the fear of wrinkling or breaking in a production line. The sample of Comparative Example 3 had a problem that it was too rigid and made a loud noise.

The samples obtained in Examples 1 to 3 each had a matte surface as shown in Fig. 2. The surface roughness of the samples of Examples 1 and 2 was formed with an embossing roll, while in Example 3 the surface roughness was imparted by means of polymer blending.

Each sample was evaluated for surface roughness, the coefficient of dynamic friction, and suitability for a production line by the following methods. The results obtained are shown in Table 4 together with the results for Comparative Example 4 (a flat polyester film having a thickness of 25 µm) and Comparative Example 5 (a flat polypropylene film having a thickness of 40 µm).

Surface Roughness

Surface roughness was evaluated in terms of the center-line mean roughness $R_a$ as provided for in JIS B 0601-1982.

Coefficient of Dynamic Friction

In accordance with JIS K-7125, measurement was made under the conditions of test sample dimensions of 50 mm by 50 mm, a sliding piece weight of 500 g, a test speed of 300 mm/min, and a mating material of stainless steel (SUS304A).

Suitability for Production Line

Suitability for a production line was evaluated based on the following criteria.

A : good
B : fair
C : poor

Table 4

| | Example | | | Comparative Example | |
|---|---|---|---|---|---|
| | 1 | 2 | 3 | 4 | 5 |
| Surface roughness (µm) | 2-3 | 5-10 | 10-15 | 0 | 0 |
| Coefficient of dynamic friction | 1.3 | 1.0 | 0.7 | 2.0 | 3.0 |
| Suitability for production line | A | A | A | B-C | C |

EXAMPLES 4 TO 6

Multilayered film samples according to the present invention were obtained which each had the layer constitution shown in Table 5. These samples were examined by the same thermomechanical analysis as described above, and the results of the determination of the average coefficients of linear expansion are shown in Table 6.

### Table 5

| | Material | Melting Point (°C) | Thickness (μm) | Total Thickness (μm) |
|---|---|---|---|---|
| **Example 4** | | | | |
| 1st layer | High-density polyethylene (HDPE) | 120 | 20 | 30 |
| 2nd layer | Low-density polyethylene (LDPE) | 100 | 10 | |
| **Example 5** | | | | |
| 1st layer | Linear polyethylene (L-LDPE) | 120 | 15 | 22 |
| 2nd layer | Low-density polyethylene (LDPE) | 100 | 7 | |
| **Example 6** | | | | |
| 1st layer | Polypropylene (random polymer) | 130 | 10 | |
| 2nd layer | Polypropylene (homopolymer) | 160 | 20 | 40 |
| 3rd layer | Polypropylene (random polymer) | 130 | 10 | |

EP 0 737 731 B1

14

Table 6

| | | Example | | |
|---|---|---|---|---|
| | | 4 | 5 | 6 |
| Average coefficient of linear expansion | | | | |
| 70-80°C | Machining direction | 0.4 | 0.6 | 0.1 |
| | Traversing direction | 0.5 | 1.2 | 0.2 |
| 80-90°C | Machining direction | 0.6 | 0.8 | 0.2 |
| | Traversing direction | 0.8 | 1.2 | 0.2 |
| 90-100°C | Machining direction | 1.1 | 1.3 | 0.2 |
| | Traversing direction | 1.2 | 2.1 | 0.4 |
| 100-110°C | Machining direction | 2.6 | 3.9 | 0.5 |
| | Traversing direction | 3.2 | 5.1 | 1.0 |
| unit: $1/°C \times 10^{-3}$ | | | | |

Other properties were examined by the same methods as described above. The results obtained are shown in Table 7.

Table 7

| | Example | | |
|---|---|---|---|
| | 4 | 5 | 6 |
| Sound pressure level, dB | | | |
|     Tearing noise | 68 | 65 | 73 |
|     Crushing noise | 69 | 63 | 75 |
|     Noise made by clumping by hand | 70 | 67 | 78 |
| Tensile strength | | | |
|     Machining direction (gf/10mm) | 510 | 420 | 600 |
|     " (N/10mm) | 5.00 | 4.12 | 5.88 |
| Traversing direction (gf/10mm) | 490 | 380 | 700 |
|     " (N/10mm) | 4.81 | 3.73 | 6.86 |
| Softness | A | A | B |

Comparative EXAMPLE 7

A resin composition obtained by incorporating 2 wt% titanium white ($TiO_2$) into a low-density polyethylene resin for improving plastic film whiteness as the only one additive ingredient was subjected to a film-forming operation. Thus, a low-density polyethylene film (thickness, 30 µm; tensile strength in the machining direction, 400 gf/10 mm (3.92 N/10mm); tensile strength in the traversing direction, 300 gf/10 mm (2.94 N/10mm)) not containing any of an antioxidant, an antistatic agent, a lubricant, and an ultraviolet absorber was produced. On one side of the low-density polyethylene film, an ultraviolet-curing silicone releasing agent was coated and irradiated with ultraviolet ray by means of a high pressure mercury lamp to thereby form a releasing agent layer having a thickness of 1 µm.

EXAMPLE 8

A sample according to the present invention was produced in the same manner as in Example 7, except that use was made of a low-density polyethylene film (thickness, 30 µm; tensile strength in the machining direction, 400 gf/10 mm (3.92 N/10mm); tensile strength in the traversing direction, 300 gf/10 mm (2.94 N/10mm)) obtained from a resin composition produced by incorporating into a low-density polyethylene resin 0.1 wt% antioxidant (2,6-di-t-butyl-p-cresol), 0.03 wt% antistatic agent (trade name, Armostat 310; manufactured by Lion Corp.), 0.1 wt% lubricant (stearamide), and 0.1 wt% ultraviolet absorber (trade name, Irganox 1010; manufactured by Ciba Geigy Ltd.).

EXAMPLE 9

The same low-density polyethylene film as in Example 8 was laminated to a polypropylene nonwoven fabric (basis weight, 30 g/m$^2$) to produce a laminated film (tensile strength in the machining direction, 800 gf/10 mm (7.85 N/10mm); tensile strength in the traversing direction, 500 gf/10 mm (4.90 N/10mm)). On the low-density polyethylene film side of the laminated film as a substrate, an ultraviolet-curing silicone releasing agent layer having a thickness of 1 μm was provided. Thus, a sample according to the present invention was obtained.

Comparative EXAMPLE 10

A sample was obtained in the same manner as in Example 7, except that use was made of a low-density polyethylene film (thickness, 30 μm; tensile strength in the machining direction, 400 gf/10 mm (3.92 N/10mm); tensile strength in the traversing direction, 300 gf/10 mm (2.94 N/10mm)) which had been obtained from the same resin composition as in Example 7 and one side of the film was subjected to embossing with an embossing roll during film formation. The sample had an ultraviolet-curing silicone releasing agent layer on the non-embossed surface of the substrate.

Comparative EXAMPLE 11

A sample was produced in the same manner as in Example 7, except that use was made of a low-density polyethylene film (thickness, 30 μm; tensile strength in the machining direction, 400 gf/10 mm (3.92 N/10mm); tensile strength in the traversing direction, 300 gf/10 mm (2.94 N/10mm)) obtained from a resin composition produced by incorporating into a low-density polyethylene resin 0.5 wt% antioxidant (2,6-di-t-butyl-p-cresol), 0.5 wt% antistatic agent (trade name, Armostat 310; manufactured by Lion Corp.), 0.5 wt% lubricant (stearamide), and 0.5 wt% ultraviolet absorber (trade name, Irganox 1010; manufactured by Ciba Geigy Ltd.).

COMPARATIVE EXAMPLE 6

A sample was produced in the same manner as in Example 11, except that use was made of a 20 μm-thick low-density polyethylene film (tensile strength in the machining direction, 200 gf/10 mm (1.96 N/10mm); tensile strength in the traversing direction 180 gf/10 mm (1.77 N/10mm)) formed from the same resin composition as in Example 11.
The average coefficients of linear expansion of the plastic films used in Examples 7 to 11 and Comparative Example 6 were determined in the same manner as the above. The results obtained are shown in Table 8.

EP 0 737 731 B1

Table 8

| | | Example | | | | | Comparative Example |
|---|---|---|---|---|---|---|---|
| | | 7* | 8 | 9 | 10* | 11* | 6 |
| Average coefficient of linear expansion | | | | | | | |
| 70–80°C | Machining direction | 0.6 | 0.6 | 0.4 | 0.6 | 0.6 | 0.8 |
| | Traversing direction | 1.2 | 1.2 | 0.7 | 1.2 | 1.2 | 1.6 |
| 80–90°C | Machining direction | 0.8 | 0.8 | 0.5 | 0.8 | 0.8 | 0.9 |
| | Traversing direction | 1.2 | 1.2 | 0.9 | 1.2 | 1.2 | 1.7 |
| 90–100°C | Machining direction | 1.2 | 1.2 | 1.0 | 1.2 | 1.2 | 1.7 |
| | Traversing direction | 2.0 | 2.0 | 1.5 | 2.0 | 2.0 | 2.8 |
| 100–110°C | Machining direction | 3.8 | 3.6 | 2.0 | 3.8 | 3.7 | 4.5 |
| | Traversing direction | 3.5 | 3.5 | 2.1 | 3.5 | 3.5 | 6.2 |

unit: $1/°C \times 10^{-3}$

* examples 7, 10, 11 are comparative examples

Other properties of the samples obtained in Examples 7 to 11 and Comparative Example 6 were examined by the following methods. The results obtained are shown in Table 9.

Change in Adhesion Strength through Contact with Releasing Agent Layer

An SIS type hot-melt pressure-sensitive adhesive heated at 80°C was applied in a thickness of 50 $\mu$m to the releasing agent layer surface of each of the samples obtained in the Examples and Comparative Examples, on a cooling roll. A polyester film having a thickness of 38 $\mu$m was then applied to the pressure-sensitive adhesive layer. Thus, samples for evaluation were produced.

The evaluation samples produced by the above-described method were allowed to stand under the conditions of (1) 23°C x 30 minutes (initial), (2) 120°C x 1 minute (after heating 1), or (3) 150°C x 1 minute (after heating 2). Thereafter, the separator sheet was peeled off, and a 50 $\mu$m-thick polyester film was applied to the pressure-sensitive adhesive layer by pressing the film by rolling a 2-kg roller forward and backward once on the film. The adhesion strength of each of the thus-obtained test pieces was measured through a tensile test in which the 38 $\mu$m-thick polyester film was peeled off with a tensile tester at a pulling rate of 300 mm/min and a peeling angle of 180°.

Appearance of Separator Sheet

After the SIS type hot-melt pressure-sensitive adhesive heated at 80°C was applied in a thickness of 50 $\mu$m, on a cooling roll,.to the releasing agent layer surface of each of the samples obtained in the Examples and Comparative Example, the state of each sample was visually examined.

## Table 9

|  | Initial Adhesion Strength | Adhesion Strength After Heating 1 | Adhesion Strength After Heating 2 | Appearance of Separator Sheet |
|---|---|---|---|---|
| Comparative Example 7 | 650 | 630 | 650 | good |
| Example 8 | 660 | 640 | 620 | good |
| Example 9 | 660 | 630 | 610 | good |
| Comparative Example 10 | 650 | 620 | 630 | good |
| Comparative Example 11 | 670 | 400 | 350 | good |
| Comparative Example 6 | 650 | 410 | 340 | shrinkage wrinkles generated |

*: unit: g/50 mm

As Table 9 shows, the samples of Examples 7 to 10 did not cause a decrease in the adhesion strength of the pressure-sensitive adhesive layer in contact with the silicone releasing agent layer. Furthermore, the samples of Examples 7 to 11 did not develop shrinkage wrinkles even when a heated hot-melt pressure-sensitive adhesive was applied thereto.

The separator, packaging material, or pressure-sensitive adhesive tape base material of the present invention has high commercial value, namely, it gives an excellent soft feeling and makes a diminished noise, and further has heat resistance and stiffness sufficient for a production line.

In the case of using a silicone releasing agent as the releasing agent layer, various pressure-sensitive adhesive members (members having a pressure-sensitive adhesive layer) in contact with the silicone releasing agent layer are prevented from suffering a decrease in adhesion performance and can retain their intact adhesion performance until use, by regulating the contents of specific additive ingredients in the plastic film substrate to a specific amount or below.

While the invention has been described in detail and with reference to specific embodiments thereof, it will be apparent to one skilled in the art that various changes and modifications can be made therein without departing from the scope thereof.

**Claims**

1. A separator, a packaging material, or a pressure sensitive adhesive tape base material comprising a substrate and a releasing agent layer formed on part or the whole of at least one side of the substrate, wherein said substrate comprises a plastic film comprising a thermoplastic resin as a main component and an antioxidant, an antistatic agent, a lubricant, and an ultraviolet absorber, the content of the antioxidant, antistatic agent, lubricant, and ultraviolet absorber each is in an amount of 0.01 to 0.4% by weight based on the amount of the plastic film, and said releasing agent layer is composed of a silicone releasing agent, and wherein when examined by thermomechanical analysis the substrate has the following values of the average coefficient of linear expansion ($1/° C \times 10^{-3}$) in the machining direction and the traversing direction for each temperature range.

| Temperature | Machining direction | Traversing direction |
|---|---|---|
| 70-80°C | ≤0.7 | ≤1.3 |
| 80-90°C | ≤0.9 | ≤1.3 |
| 90-100°C | ≤1.4 | ≤2.2 |
| 100-110°C | ≤4.0 | ≤5.2 |

2. The separator, packaging material, or pressure-sensitive adhesive tape base material of claim 1, wherein said substrate is composed of at least two layers in which one layer is in contact on one or each side with a heat-sealable layer having a melting point lower by at least 5°C than the melting point of said one layer.

3. The separator, packaging material, or pressure-sensitive adhesive tape base material of claim 1, wherein said substrate comprises a plastic film comprising a thermoplastic polyolefin resin as a main component.

4. The separator, packaging material, or pressure-sensitive adhesive tape base material of claim 1, which has a sound pressure level of 80 dB or lower.

5. The separator, packaging material, or pressure-sensitive adhesive tape base material of claim 1, wherein said substrate has a thickness of 500 μm or smaller.

6. The separator, packaging material, or pressure-sensitive adhesive tape base material of claim 1, which has a tensile strength in the machining direction of 300 gf/10 mm (2.94 N/10 mm) or higher and a tensile strength in the traversing direction of 200 gf/10 mm (1.96 N/10 mm) or higher.

7. The separator, packaging material, or pressure sensitive adhesive tape base material of claim 1 wherein the substrate is a film comprising a blend of thermoplastic resins selected from ultralow-density, low-density, linear low-density, medium-density, and high-density polyethylenes.

8. Use of the packaging material of claim 1 as a packaging sheet for a sanitary napkin.

## EP 0 737 731 B1

**Patentansprüche**

1.  Trennteil, Verpackungsmaterial oder druckempfindliches Klebeband-Grundmaterial, das ein Substrat und eine an einem Teil oder der Gesamtheit mindestens einer Seite des Substrats ausgebildete Trennmittel-Lage umfasst, wobei das Substrat einen Kunststofffilm mit einem thermoplastischen Harz als Hauptkomponente und ein Antioxidans, ein Antistatikum, ein Schmiermittel und einen Ultraviolett-Absorber umfasst, wobei der Anteil des Antioxidans, des Antistatikums, des Schmiermittels und des Ultraviolett-Absorbers jeweils in Höhe von 0,01 bis 0,4 Gew.-%, bezogen auf die Menge des Kunststofffilms beträgt, und die Trennmittellage aus einem Silikon-Lösemittel besteht, und wobei unter Prüfung durch thermomechanische Analyse das Substrat die folgenden Werte des durchschnittlichen linearen Ausdehnungskoeffizienten (1/°C x10$^{-3}$) in der Bearbeitungsrichtung bzw. der Querrichtung für jeden Temperaturbereich aufweist:

| Temperatur | Bearbeitungsrichtung | Querrichtung |
|---|---|---|
| 70-80°C | ≤ 0,7 | ≤ 1,3 |
| 80-90°C | ≤ 0,9 | ≤ 1,3 |
| 90-100°C | ≤ 1,4 | ≤ 2,2 |
| 100-110°C | ≤ 4,0 | ≤ 5,2 |

2.  Trennteil, Verpackungsmaterial oder druckempfindliches Klebeband-Grundmaterial nach Anspruch 1, bei dem das Substrat aus mindestens zwei Lagen zusammengesetzt ist, von denen eine Lage an einer Seite oder jeder Seite mit einer heißsiegelbaren Lage in Berührung ist, die einen um mindestens 5°C niedrigeren Schmelzpunkt als dem Schmelzpunkt der einen Lage aufweist.

3.  Trennteil, Verpackungsmaterial oder druckempfindliches Klebeband-Grundmaterial nach Anspruch 1, bei dem das Substrat einen thermoplastisches Polyolefinharz als Hauptkomponente umfassenden Kunststofffilm umfasst.

4.  Trennteil, Verpackungsmaterial oder druckempfindliches Klebeband-Grundmaterial nach Anspruch 1, das einen Schalldruckpegel von 80 dB oder niedriger aufweist.

5.  Trennteil, Verpackungsmaterial oder druckempfindliches Klebeband-Grundmaterial nach Anspruch 1, bei dem das Substrat eine Dicke von 500 μm oder weniger hat.

6.  Trennteil, Verpackungsmaterial oder druckempfindliches Klebeband-Grundmaterial nach Anspruch 1, das eine Zugfestigkeit in der Bearbeitungsrichtung von 2,94 N/10 mm (300 gf/10 mm) oder höher und eine Zugfestigkeit in der Querrichtung von 1,96 N/10 mm (200 gf/10 mm) oder höher hat.

7.  Trennteil, Verpackungsmaterial oder druckempfindliches Klebeband-Grundmaterial nach Anspruch 1, bei dem das Substrat ein eine Mischung von thermoplastischen Harzen, ausgewählt aus ultraniedrigdichten, niedrigdichten, linear niedrigdichten, mitteldichten und hochdichten Polyethylenen, umfassender Film ist.

8.  Verwendung des Verpackungsmaterials nach Anspruch 1 als Verpackungsschicht für eine Damenbinde.

**Revendications**

1.  Séparateur, matériau de conditionnement ou matériau de base de ruban adhésif sensible à la pression, comprenant un substrat et une couche d'agent de séparation formée sur une partie ou la totalité d'un côté au moins du substrat, dans lequel le substrat comprend un film de matière plastique qui contient une résine thermoplastique comme principal ingrédient et un antioxydant, un agent antistatique, un lubrifiant et un agent absorbant les ultraviolets, la teneur en antioxydant, en agent antistatique, en lubrifiant et en agent absorbant les ultraviolets étant, pour chacun, une quantité comprise entre 0,01 et 0,4 % en poids par rapport à la quantité du film de matière plastique, et la couche d'agent de séparation est composée d'un agent de séparation de silicone, et dans lequel, lors d'un examen par analyse thermomécanique, le substrat a les valeurs suivantes du coefficient moyen de dilatation linéaire (1/°C. 10$^{-3}$) dans la direction de la machine et dans la direction transversale pour chaque plage de températures :

| Température | Direction de la machine | Direction transversale |
|---|---|---|
| 70-80 °C | ≤ 0,7 | ≤ 1,3 |
| 80-90 °C | ≤ 0,9 | ≤ 1,3 |
| 90-100 °C | ≤ 1,4 | ≤ 2,2 |
| 100-110 °C | ≤ 4,0 | ≤ 5,2 |

**2.** Séparateur, matériau de conditionnement ou matériau de base de ruban adhésif sensible à la pression selon la revendication 1, dans lequel le substrat est composé d'au moins deux couches, une première couche étant au contact d'un côté ou de chaque côté d'une couche thermosoudable ayant une température de fusion inférieure d'au moins 5 °C à la température de fusion de la première couche.

**3.** Séparateur, matériau de conditionnement ou matériau de base de ruban adhésif sensible à la pression selon la revendication 1, dans lequel le substrat est un film de matière plastique contenant une résine de polyoléfine thermoplastique comme principal ingrédient.

**4.** Séparateur, matériau de conditionnement ou matériau de base de ruban adhésif sensible à la pression selon la revendication 1, qui a un niveau de pression sonore inférieur ou égal à 80 dB.

**5.** Séparateur, matériau de conditionnement ou matériau de base de ruban adhésif sensible à la pression selon la revendication 1, dans lequel le substrat a une épaisseur inférieure ou égale à 500 μm.

**6.** Séparateur, matériau de conditionnement ou matériau de base de ruban adhésif sensible à la pression selon la revendication 1, qui possède une résistance à la traction dans la direction de la machine de 2,94 N/10 mm (300 gf/10 mm) ou plus et une résistance à la traction dans la direction transversale supérieure ou égale à 1,96 N/10 mm (200 gf/10 mm).

**7.** Séparateur, matériau de conditionnement ou matériau de base de ruban adhésif sensible à la pression selon la revendication 1, dans lequel le substrat est un film comprenant un mélange de résines thermoplastiques choisies parmi les polyéthylènes de densité extrêmement faible, de faible densité, linéaire de faible densité, de densité moyenne et de haute densité.

**8.** Application du matériau de conditionnement selon la revendication 1 à une feuille de conditionnement d'une serviette hygiénique.

Fig. 1

Fig. 2

Fig. 3

Fig. 4

Fig. 5

Fig. 6

Fig. 7

Fig. 8